# EUROPÄISCHE PATENTSCHRIFT

(11) **EP 1 109 503 B1**
(45) Veröffentlichungstag und Bekanntmachung des Hinweises auf die Patenterteilung: **11.03.2009**
(21) Anmeldenummer: 99946089.2
(22) Anmeldetag: 01.09.1999
(51) Int. Cl.: A61B 18/00

(54) **HOCHFREQUENZEINRICHTUNG ZUR ERZEUGUNG EINES PLASMABOGENS FÜR DIE BEHANDLUNG BIOLOGISCHEN GEWEBES**
HIGH-FREQUENCY DEVICE FOR GENERATING A PLASMA ARC FOR THE TREATMENT OF BIOLOGICAL TISSUE
DISPOSITIF HAUTE FREQUENCE PERMETTANT DE PRODUIRE UN ARC DE PLASMA DESTINE AU TRAITEMENT D'UN TISSU BIOLOGIQUE

(30) Priorität: 01.09.1998 DE 19839826
(43) Veröffentlichungstag der Anmeldung: 27.06.2001
(73) Patentinhaber: Lindenmeier, Heinz, Prof. Dr.-Ing., 82152 Planegg (DE); Fastenmeier, Karl, 81739 München (DE)
(72) Erfinder: Lindenmeier, Heinz, Prof. Dr.-Ing., 82152 Planegg (DE); Fastenmeier, Karl, 81739 München (DE)
(74) Vertreter: Bohnenberger, Johannes
(86) Internationale Anmeldenummer: PCT/EP1999/006437
(87) Internationale Veröffentlichungsnummer: WO 2000/012019

(56) Entgegenhaltungen:
- WO-A-98/35618
- US-A- 3 903 891

## Beschreibung

Die Erfindung betrifft eine Hochfrequenzeinrichtung zur Erzeugung eines Plasmabogens für die Behandlung von biologischem Gewebe nach dem Oberbegriff des Anspruches 1.

Hochfrequenzeinrichtungen dieser Art sind bereits bekannt aus DE-OS 37 10 489, der EP 0 353 178 A2 sowie der WO 93/01758. Die Oberflächenbehandlung von menschlichem Gewebe kann im Milieu unterschiedlicher Gase erfolgen. Häufig wird zur besseren räumlichen Ausformung des Plasmastrahls ein konzentriert auf das zu behandelnde Gewebe gerichteter Strahl eines Gases eingesetzt, dessen Ionisationsfeldstärke niedriger ist als die des Gases im umgebenden Milieu.

Bezüglich der Zuführung von Hochfrequenzenergie zur Erzeugung eines Plasmastrahls ergeben sich in jedem Fall zwei grundlegende Problemkreise. Diese bestehen zum einen in der sicheren Zündung des Plasmastrahls nach einem Erlöschen während der Applikation und vor dem Ersteinsatz und zum zweiten in der Dosierung der HF-Stromstärke im gezündeten Plasmastrahl bezüglich der für die medizinische Anwendung geeigneten Intensität.

Das letztgenannte Problem wird in der DE-OS 37 10 489 und in der EP 0 353 178 A2 ausführlich gewürdigt und mit einer hoch komplizierten Einrichtung zur Regelung und Steuerung der dem Plasma zugeführten Energie gelöst. Die Steuerstrecke ist hierbei durch eine HF-Treiberschaltung gebildet, welche einen Ausgangsschwingkreis mit einer vorbestimmten Frequenz auflädt und wobei der Ausgangsschwingkreis sich mit seiner Resonanzfrequenz entlädt, indem er elektrische Energie an das Gewebe abgibt. Mit Hilfe von Frequenzteilern werden Frequenzsignale gewonnen, um das Anlegen und die Dauer der Speise- oder Treiberimpulse zu steuern, welche an den Ausgangsschwingkreis abgegeben werden. Die HF-Treiberschaltung nimmt somit innerhalb eines komplizierten Regelkreises eine Schaltfunktion wahr. Um mit einer derartigen Einrichtung eine sichere Zündung mit wesentlich über der Brennspannung des Plasmastrahls liegender Elektrodenspannung herbeizuführen, ist es somit zwangsweise nötig, vergleichsweise zeitlich lange Treiberimpulse einzustellen. Nach Zündung des Plasmas durch das Regelsystem muß in - durch die Regelzeitkonstante vorgegebener - kurzer Zeit eine Einregelung auf die für die medizinische Anwendung passende Intensität erfolgen. WO-A-9835618 offenbart eine Hochfrequenzeinrichtung mit den Merkmalen des Oberbegriffs von Anspruch 1.

De Erfindung liegt deshalb die Aufgabe zugrunde, eine verbesserte Hochfrequenzeinrichtung zur Erzeugung eines Plasmabogens nach dem Oberbegriff des Anspruches 1 mit einer einfachen Schaltung anzugeben.

Diese Aufgabe wird durch eine Hochfrequenzeinrichtung mit den Merkmalen des Anspruches 1 gelöst.

Hierbei liegt im Zustand des ungezündeten Plasmas die Hochfrequenzspannung an der Elektrode nennenswert über der zur Zündung des Plasmabogens notwendigen Spannung und besitzt im gezündeten Zustand des Plasmas der Hochfrequenzstrom in der Plasmastrecke die für die medizinische Anwendung passende Intensität, und ebenso steigt bei Erlöschen des Plasmabogens während der Applikation die Hochfrequenzspannung an der Elektrode wiederum über die für die Zündung des Plasmabogens notwendige Spannung an, so daß eine Wiederzündung automatisch gegeben ist.

Ein wesentlicher Vorteil der erfindungsgemäßen Lösung besteht darin, daß ohne Vorsehen einer zusätzlichen Regelschaltung, also mit geringem gerätetechnischen Aufwand, eine stabile und präzise steuerbare Plasmastrecke zwischen der Elektrode und der gewünschten Eintrittsstelle des Plasmabogens ins Gewebe erhalten werden kann. Dadurch lassen sich der Methode zusätzliche Anwendungen (etwa auf dem Gebiet der Neurochirurgie und bei Läsionen der Schleimhaut in der Speiseröhre erschließen, bei denen die HF-Chirurgie bislang aufgrund der großen Gefahr der thermischen Schädigung benachbarter Gewebsbereiche nicht angewandt wird

Ein weiterer wesentlicher Vorteil besteht darin, daß bei der vorgeschlagenen Lösung die Hochfrequenzenergie im wesentlichen ausschließlich am bzw. im Gewebe in Wärme umgesetzt wird, da zur Regelung keine Wirkwiderstände eingesetzt werden. Damit entfällt die Notwendigkeit von großen Kühlflächen oder einer aktiven Kühlung mittels Lüftern (die im klinischen Bereich höchst unerwünscht ist), was erhebliche Einsparungen an gerätetechnischem Aufwand, Gerätevolumen und Energie während des Betriebes mit sich bringt und für eine erhöhte Akzeptanz des Gerätes bei den Anwendern im klinischen Bereich sorgt.

Ausführungsbeispiele der Erfindung, die in der Zeichnung dargestellt sind, werden im folgenden näher beschrieben. Im einzelnen zeigen:
Fig.1 eine Hochfrequenzeinrichtung zur Erzeugung eines Plasmabogens nach einer Ausführungsform der Erfindung,
Fig. 2a eine Hochfrequenzeinrichtung gem. Fig. 1 mit einem aus einem Kondensator und der Zuleitungskapazität gebildeten Blindwiderstand 9a und einem aus einer Induktivität gebildeten Blindwiderstand 9b,
Fig. 2b eine Hochfrequenzeinrichtung gem. Fig. 1 mit einem seriellen induktiven Blindwiderstand 9b,
Fig. 2c eine Hochfrequenzeinrichtung gem. Fig. 1 mit einem aus der Parallelschaltung einer Parallelinduktivität mit Parallelkapazität und der Kapazität der Zuleitung gebildeten ersten Blindwiderstand 9a und einer Serienkapazität als zweitem Blindwiderstand 9b,
Fig. 3 eine Hochfrequenzeinrichtung gem. Fig. 2c mit einer im Gehäuse des Hochfrequenzgenerators befindlichen Serienkapazität als zweitem Blindwiderstand 9b,
Fig. 4a eine Hochfrequenzeinrichtung gem. Fig. 3 mit einer isoliert unipolar zum Patienten und zur Elektrode ausgeführten Zuleitung zur Operationsstelle;
Fig. 4b eine Hochfrequenzeinrichtung gem. Fig. 3 mit einer Zuleitung zur Elektrode mit hochfrequenzmäßig geerdetem koaxialen Schirmleiter mit Isolationsschicht,
Fig. 4c eine Hochfrequenzeinrichtung gem. Fig. 3 mit einer Zuleitung und einer an ihrem distalen Ende befindlichen inneren Masseelektrode, welche den koaxialen Schirmleiter mit dem menschlichen Gewebe galvanisch verbindet,
Fig. 4d eine Hochfrequenzeinrichtung gem. Fig. 3 mit einer Zuleitung gemäß Fig. 4b und mit einem Edelgasstrom, welcher durch das Innere des koaxialen Schirmleiters zur Elektrode und dem Operationsort am menschlichen Gewebe zugeführt wird,
Fig. 4e eine Hochfrequenzeinrichtung gem. Fig. 3 mit einer Zuleitung gemäß Fig. 4c und einem Edelgasstrom, welcher durch das Innere des koaxialen Schirmleiters zur Elektrode und dem Operationsort am menschlichen Gewebe zugeführt wird,
Fig. 4f eine Hochfrequenzeinrichtung gem. Fig. 3 mit einer unipolaren Zuleitung gemäß Fig. 4a, jedoch mit einem diese umgebenden isolierendem Schlauch, durch dessen Inneres der Edelgasstrom zur Elektrode und zum Operationsort am menschlichen Gewebe zugeführt wird,
Fig. 5a eine Hochfrequenzeinrichtung gemäß Fig. 3 mit einer ersten Kondensatorbatterie und einer zweiten Kondensatorbatterie,
Fig. 5b eine Hochfrequenzeinrichtung gemäß Fig. 2a, jedoch mit einer Kondensatorbatterie und einer Batterie von Induktivitäten,
Fig. 6a eine Hochfrequenzeinrichtung mit einem Transformator mit wählbarem Übersetzungsverhältnis,
Fig. 6b eine Hochfrequenzeinrichtung mit einem Hochfrequenzgenerator mit niedrigem Innenwiderstand, welcher aus einer hochohmigen HF-Stromquelle mit Resonanztransformation gebildet ist,
Fig. 7 eine Hochfrequenzeinrichtung gemäß Fig. 3, deren Hochfrequenzspannungsquelle mit Innenwiderstand durch eine als Schalter wirkende komplementäre Transistorstufe mit Schalttransistoren gebildet ist,
Fig. 8 eine Hochfrequenzeinrichtung nach Fig. 1 mit einem Generator auf vorgegebener Frequenz und einer Resonanzschaltung,
Fig. 9 eine Hochfrequenzeinrichtung gemäß Fig. 8 zur Einstellung des für die medizinische Anwendung notwendigen Plasmastroms mit Hilfe einer Patientenstromauskopplung, einem Patientenstromindikator und eines nachgeschalteten Patientenstromreglers,
Fig. 10 eine Hochfrequenzeinrichtung gemäß Fig. 8 bzw. Fig. 9 mit einem einstellbaren ersten Blindwiderstand 9a,
Fig. 11 eine beispielhaft gestaltete variable Induktivität zur trägheitslosen Abstimmung der Resonanz für Anordnungen gemäß den Figuren 8 bis 10,
Fig. 12 ein Beispiel einer Anordnung mit Abstimmung und Einstellung der Intensität des Plasmastroms.

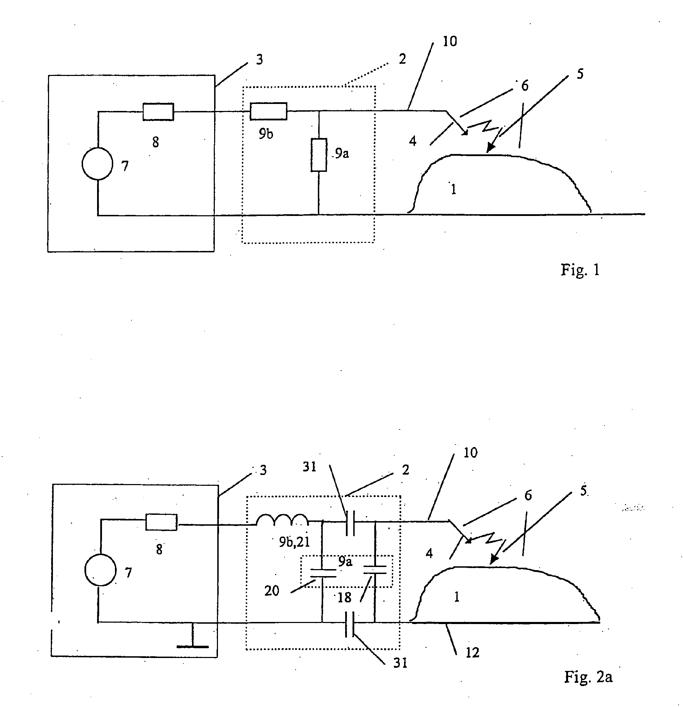

Die Wirkungsweise der vorliegenden Erfindung wird zunächst anhand der Anordnung in Fig. 1 geschildert.

Diese Anordnung umfaßt einen Hochfrequenzgenerator 3, welcher eine Hochfrequenzspannungsquelle 7 mit einem Innenwiderstand 8 beinhaltet. An diesen Hochfrequenzgenerator 3, welcher periodische Hochfrequenzschwingungen abgibt, ist eine Serienschaltung aus einem zweiten Blindwiderstand 9b und einem ersten Blindwiderstand 9a angeschlossen. Die (erhöhte) Resonanzspannung, die am ersten Blindwiderstand 9a entsteht, bildet sich nach Maßgabe der unvermeidbaren Verluste in den beiden Blindwiderständen in Verbindung mit dem durch den Innenwiderstand 8 eingebrachten Bedämpfungswiderstand aus. Ist diese Spannungsamplitude größer als die für die Zündung eines Plasmabogens 5 zwischen einer Elektrode 4 und dem menschlichen Gewebe 1 notwendige Spannung, so wird sich der Plasmabogen 5 unmittelbar ausbilden.

Die Brennspannung der Plasmastrecke 6 liegt bekanntlich unter der Zündspannung. Infolge der durch den Plasmastrom bewirkten Bedämpfung der Resonanzanordnung stellt sich nach Zündung ein Plasmastrom ein, dessen Intensität ganz wesentlich von der Spannung der Hochfrequenzspannungsquelle 7, dem Wert des zweiten Blindwiderstands 9b und der Bedämpfung des ersten Blindwiderstands 9a und des zweiten Blindwiderstands 9b abhängt.

Es ist somit vorteilhaft, den Innenwiderstand 8 im Vergleich zu den Blindwiderstandswerten des ersten Blindwiderstands 9a bzw. des zweiten Blindwiderstands 9b bei der Resonanzfrequenz klein zu gestalten.

Für den Sonderfall eines vernachlässigbar kleinen niederohmigen Innenwiderstands 8 und eines im Vergleich zur Bedämpfung durch den Plasmastrom vernachlässigbaren Dämpfungsverlusts im ersten Blindwiderstand 9a und im zweiten Blindwiderstand 9b und für den Fall, daß die Brennspannung des Plasmabogens 5 wesentlich unter der Zündspannung liegt, wird der Plasmastrom in der Hauptsache durch die Größe des zweiten Blindwiderstands 9b bestimmt, wenn die Spannung der Hochfrequenzspannungsquelle 7 wesentlich größer ist als die Brennspannung des Plasmabogens 5.

Der wesentliche Vorteil, der mit der Erfindung einhergeht, ist die unmittelbare Wiederzündung des Plasmabogens, wenn dieser durch Manipulationen bei einer Operation erloschen ist. Denn bei Erlöschen des Plasmastroms und bei Wegfall der durch diesen bedingten Bedämpfung der Resonanzanordnung steigt die Spannung am ersten Blindwiderstand 9a und damit an der Plasmastrecke 6 wieder auf den Wert an, der bei schwach bedämpfter Resonanz vorliegt, wodurch die Plasmastrecke selbsttätig wieder zündet. Eine Regeleinrichtung ist somit hierfür nicht erforderlich.

Die nach dem Erlöschen für die Wiederzündung benötigte Zeit liegt in der Größenordnung einiger Perioden der Hochfrequenzschwingung, welche bei kleiner Gesamtdämpfung im Vergleich zu der vom Operateur wahrnehmbaren Zeit extrem kurz ist.

Ein weiterer Vorteil der Erfindung liegt darin, daß nicht die Anforderung an den Hochfrequenzgenerator gestellt werden muß, eine sinusförmige Spannung abzugeben. Die Spannung muß lediglich periodisch sein und einen nennenswerten spektralen Anteil auf der Resonanzfrequenz der Resonanzschaltung 2 besitzen. Dennoch bewirkt die Resonanzschaltung 2, daß der Strom im Resonanzkreis, und damit der Patientenstrom, nach Maßgabe der Selektivität weitgehend sinusförmig ist.

In den Figuren 2a bis 2c sind beispielhaft verschiedene vorteilhafte Realisierungsmöglichkeiten für den Hochfrequenzgenerator 3 mit nachgeschalteter Resonanzschaltung 2 angegeben.

In Fig. 2a wird als erster Blindwiderstand 9a ein Parallelkondensator 20 geschaltet, dem die wirksame Kapazität 18 parallelgeschaltet ist, welche zwischen der Zuleitung zur Elektrode 4 und der Umgebung bzw. dem menschlichen Gewebe 1 gebildet ist. Als zweiter Blindwiderstand 9b wird eine Induktivität 21 verwendet, welche bei reellem Innenwiderstand 8 die Resonanzbedingung erfüllt. Für den Fall eines mit einem Blindwiderstandsanteil versehenen Innenwiderstandes 8 wird die Resonanzschaltung 2 zusammen mit diesem Innenwiderstand 8 die Resonanzbedingung bei einer geringfügig veränderten Frequenz erfüllen, was das Wesen der Anordnung nicht verändert.

Aus Sicherheitsgründen ist im Hochfrequenzgenerator 3 ein Masseanschluß 11 vorhanden. Sicherheitsvorschriften fordern ferner galvanische Trennungen zwischen dem Patienten und der Gehäusemasse. Deshalb sind in Fig. 2a galvanische Trennkondensatoren 31 gezeigt, welche jedoch mit ihrem Kapazitätswert so groß gewählt sind, daß sie das Hochfrequenzverhalten nicht beeinflussen. Deshalb sind sie in den folgenden Figuren nicht überall dargestellt.

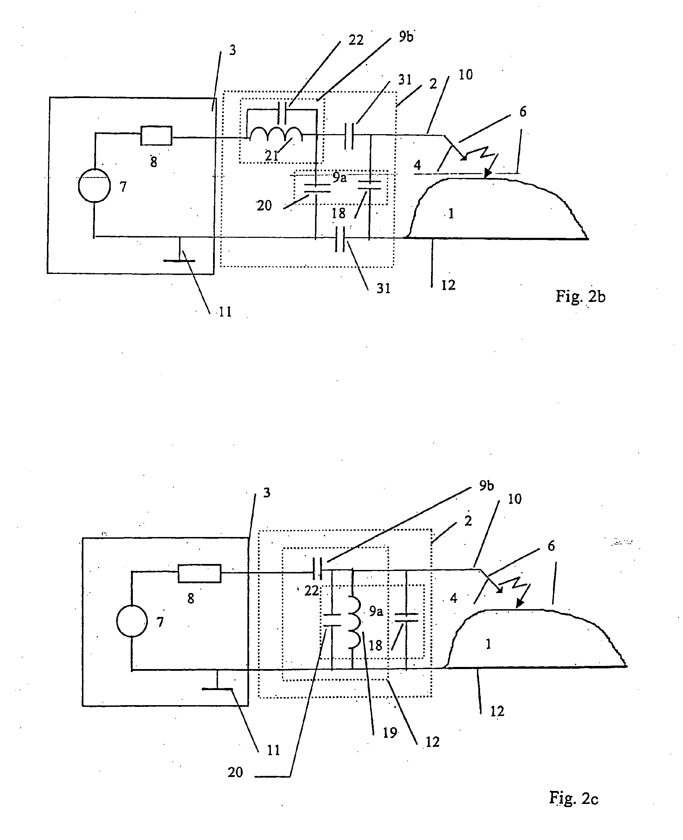

In Fig. 2b ist parallel zur Serieninduktivität 21 eine Serienkapazität 22 gezeigt, welche als Wicklungskapazität der Serieninduktiviät 21 wirksam sein kann oder zum Zwecke der Feinabstimmung zusätzlich geschaltet ist.

In einer vorteilhaften Ausgestaltung der Erfindung wird, wie in Fig. 2c dargestellt, eine Parallelinduktivität 19 mit der häufig unvermeidbaren bzw. zur Abstimmung verwendeten Parallelkapazität 20 zur Bildung des ersten Blindwiderstands 9a verwendet, welcher insgesamt induktiv wirkt. Damit ist der Vorteil verbunden, daß die Induktivität im Parallelzweig verwendet ist und somit einseitig mit dem Masseanschluß verbunden werden kann. Der zweite Blindwiderstand 9b ist dann als Kondensator ausgeführt. Es besteht eine Verbindung zwischen dem menschlichen Gewebe 1 und dem das Erdpotential führenden Masseanschluß 11 des Hochfrequenzgenerators 3.

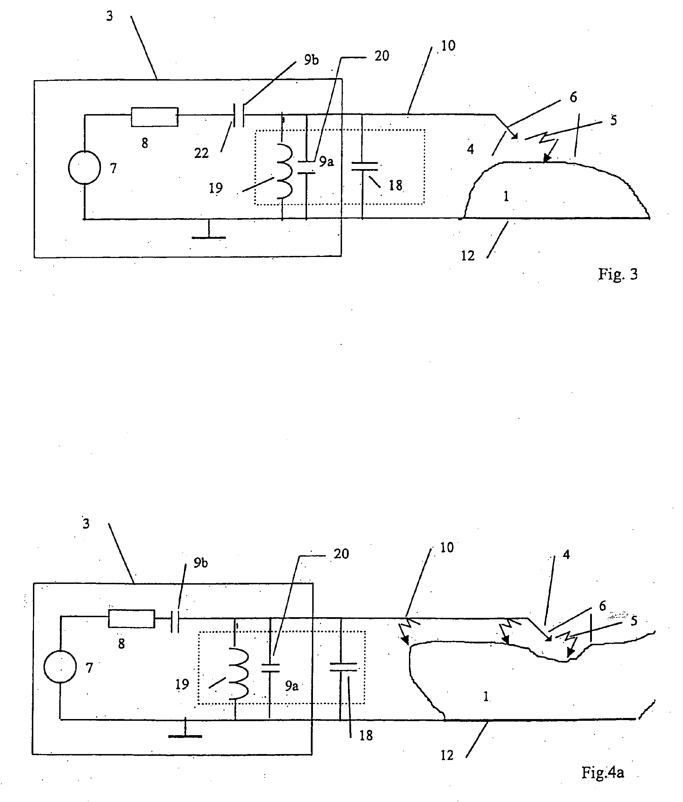

In einer weiteren vorteilhaften Ausgestaltung der Erfindung sind, wie in Fig. 3 dargestellt, die wesentlichen Elemente der Resonanzschaltung 2 im Gehäuse des Hochfrequenzgenerators 3 untergebracht. Dieses umfaßt eine zur Plasmastrecke 6 parallel liegende Parallelinduktivität 19. Lediglich die wirksame Kapazität 18, welche die Kapazität der Zuleitung 10 zur Plasmastrecke 6 repräsentiert, befindet sich als Teil des ersten Blindwiderstands 9a außerhalb des Hochfrequenzgenerators 3.

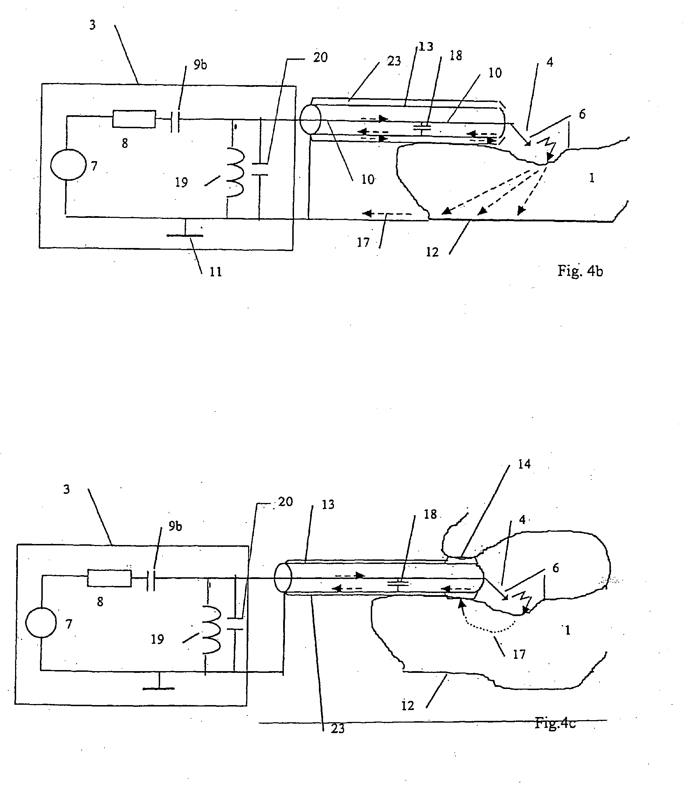

In Fig. 4a ist das Problem aufgezeigt, daß es bei nicht isolierter Zuleitung 10 zu unerwünschten Plasmazündungen zum Gewebe neben dem Plasmabogen 5 kommen kann. Dies ist in Fig. 4a durch Pfeile gekennzeichnet. In einer vorteilhaften Ausführung der Erfindung wird deshalb die Zuleitung 10 mit einem koaxialen Schirmleiter 13 umgeben, welcher mit einer Isolationsschicht 23 überzogen ist. Dies ist in Fig. 4b dargestellt. Die gestrichelten Pfeile beschreiben hierbei die Vor- bzw. Rückströme 17, die sich bei dieser Anordnung während der Applikation ausbilden. Durch den koaxialen Schirmleiter 13 wird die Ausbildung von Plasmabögen zwischen der Zuleitung 10 und dem menschlichen Gewebe 1 verhindert. Der Plasmastrom fließt als Gewebestrom im menschlichen Gewebe 1 zur äußeren HF-Masseeleektrode 12 am Patienten und wird zum Hochfrequenzgenerator 3 zurückgeführt. Auf der Außenhaut des Schirmleiters fließen Ströme, welche die Operation nicht beeinflussen. Auf diese Weise ist sichergestellt, daß sich der Plasmabogen 5 ausschließlich zwischen der Elektrode 4 und dem menschlichen Gewebe 1 ausbildet.

Bei einigen Operationsarten kann es schädlich sein, wenn der Rückstrom des Plasmabogens 5 im menschlichen Gewebe 1 zur äußeren HF-Masseelektrode 12 unkontrolliert diffus erfolgt. In solchen Fällen ist es im Hinblick auf die örtliche Selektivität der Ausbildung des Plasmabogens 5 in Verbindung mit einer örtlich begrenzten Stromrückführung im menschlichen Gewebe 1 zum koaxialen Schirmleiter 13 vorteilhaft, den koaxialen Schirmleiter 13 an seinem distalen Ende mit einer inneren HF-Masseelektrode 14 zu versehen, welche dort mit dem menschlichen Gewebe 1 leitend in Verbindung steht. Eine entsprechende Anordnung zeigt Fig. 4c. Der Rückstrom 17 kann sich dabei örtlich nur im begrenzten Umfeld des Operationsorts ausbilden. Der Rückstrom erfolgt dann auf der Innenseite des koaxialen Schirmleiters 13.

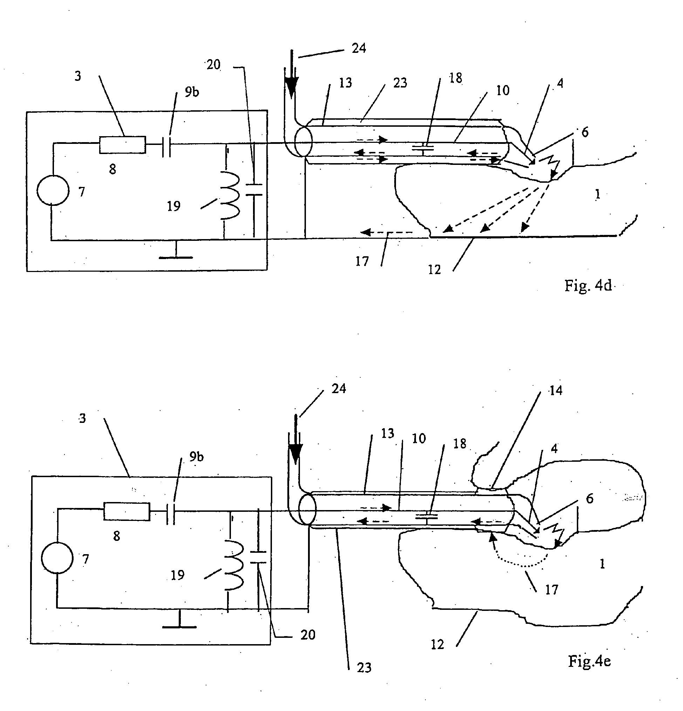

Eine weitere vorteilhafte Anwendung der Erfindung ergibt sich in Verbindung mit einem dem Operationsort gezielt zugeführten Edelgasstrom 24. Die Zuführung kann auf vorteilhafte Weise in einem Hohlraum innerhalb des koaxialen Schirmleiters 13 erfolgen, wie es in Fig. 4d dargestellt ist. Durch die Applikation des Edelgasstroms 24 wird die Ionisationsenergie stark herabgesetzt, wodurch auch die Zündspannung für die Plasmastrecke 6 wesentlich verringert wird und eine Ionisation außerhalb des Edelgasstroms nicht erfolgt. Als Edelgas eignet sich z.B. Argon, welches vielfach zur Anwendung kommt. Der Edelgasstrom 24, welcher am distalen Ende gezielt zur Unterstützung der Ausbildung des Plasmabogens 5 zugeführt ist, erlaubt dabei eine hohe Selektivität bezüglich der räumlichen Ausdehnung des Plasmabogens 5.

Aus gleichem Grund wird - wie in Fig. 4e gezeigt - bei einer Anordnung gemäß Fig. 4c ein Edelgasstrom 24 innerhalb des Schirmleiters 13 zugeführt. Alternativ kann der Edelgasstrom auch in einem hohlen Innenleiter zugeführt werden.

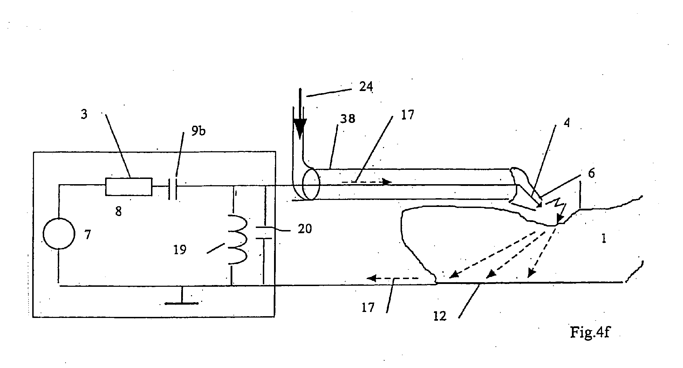

Im Gegensatz hierzu wird bei der Anordnung in Fig. 4f mit unipolarer Zuleitung kein koaxialer Schirmleiter 13 verwendet, sondern an dessen Stelle ein isolierender Schlauch 38, innerhalb dessen der Edelgasstrom 24 zur Elektrode 4 geleitet wird.

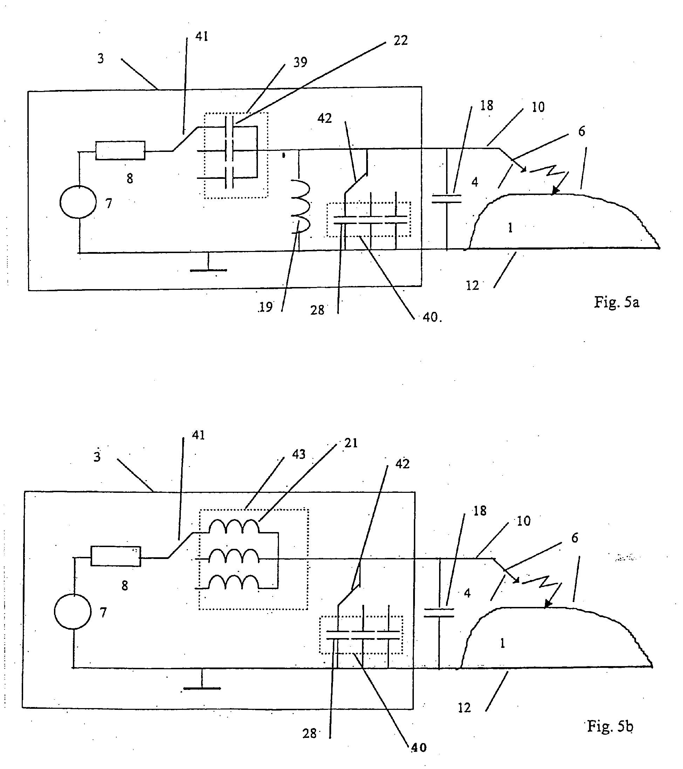

Bei der in Fig. 5a gezeigten bevorzugten Ausführung ist die Kondensatorbatterie 39 beispielsweise aus Kapazitäten von 50 pF, 100 pF und 200 pF und die Kondensatorbatterie 40 aus Kapazitäten von 16 pF, 116 pF und 216 pF gebildet, der Kondensator 18 hat eine Kapazität von 250 pF und die Induktivität 19 einen Wert von ca. 500 µH, und der Hochfrequenzgenerator wird bei einer Frequenz von 330 kHz betrieben. Die von der Hochfrequenzspannungsquelle 7 abgegebene Spannung wird im Bereich zwischen 40 und 300 V geregelt, und es kann in einem Abstandsbereich der Elektrode 4 zum menschlichen Gewebe 1 von 1 bis etwa 25 mm gearbeitet werden.

In Fig. 5a und 5b sind verschiedene Möglichkeiten der Einstellung der Resonanzbedingung sowie der Intensität des Plasmastroms während der Operation aufgezeigt. In einer besonders vorteilhaften Anordnung sind gemäß Fig. 5a eine erste Kondensatorbatterie 39 und eine zweite Kondensatorbatterie 40 vorgesehen, aus welchen jeweils mit Hilfe eines ersten Schalters 41 eine Serienkapazität 22 bzw. mit Hilfe eines zweiten Schalters 42 ein geeigneter Kondensator 28 ausgewählt werden. Durch geeignete Kombination der Schalterstellungen und geeignete Wahl der Kapazitätswerte werden damit für einen festen Induktivitätswert der Parallelinduktivität 19 sowohl die Resonanzbedingung für die Frequenz der Hochfrequenzspannungsquelle 7 als auch die Intensität des Plasmabogens 5 bei der gegebenen Spannung der Hochfrequenzspannungsquelle 7 eingestellt.

Als eine weitere Möglichkeit wird in Fig. 5b der zweite Blindwiderstand 9b als eine Serieninduktivität 21 realisiert, wobei mit Hilfe eines ersten Schalters 41 diese Induktivität aus einer Batterie von Induktivitäten 43 ausgewählt wird. Der erste Blindwiderstand 9a wird als geeigneter Kondensator 28 mit Hilfe eines zweiten Schalters 42 aus einer Kondensatorbatterie 40 ausgewählt.

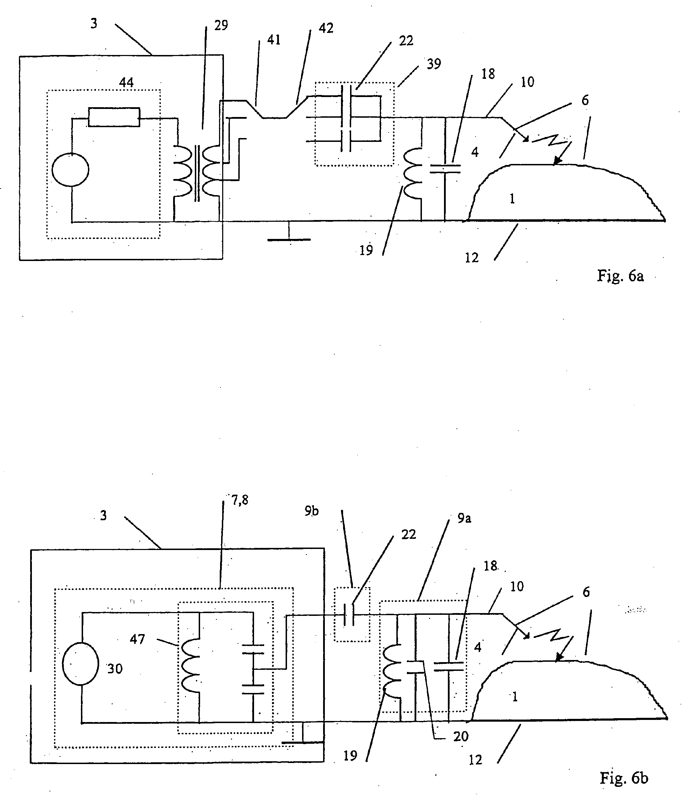

Vielfach ist ein vorgegebener Hochfrequenzgenerator 44 zur Bildung einer Hochfrequenzeinrichtung zur Erzeugung eines Plasmabogens vorhanden, welcher auch für andere Applikationen bestimmt ist. In diesem Falle ist es, wie in Fig. 6a dargestellt, vorteilhaft, an einem Transformator 29, welcher die Einstellung mehrerer Übersetzungsverhältnisse ermöglicht, mit Hilfe eines ersten Schalters 41 das Übersetzungsverhältnis einzustellen. Die Auswahl einer Serienkapazität 22 aus einer ersten Kondensatorbatterie 39 erfolgt dann mit Hilfe eines zweiten Schalters 42. Ist die Hochfrequenzspannungsquelle nur als hochohmige HF-Stromquelle verfügbar, so kann diese in eine Hochfrequenzspannungsquelle 7 mit niederohmigem Innenwiderstand 8 umgewandelt werden. Dies erfolgt, wie in Fig. 6b gezeigt, mit Hilfe einer Resonanztransformationsschaltung 47. Der in diesem Bild mit 3 gekennzeichnete Hochfrequenzgenerator bildet dann an seinem Ausgang die Hochfrequenzspannungsquelle 7 mit niederohmigem Innenwiderstand 8.

Die Signalform, welche bei der vorliegenden Erfindung zur Anwendung kommt, erfüllt lediglich die Voraussetzung der Periodizität. Als besonderer Vorteil der Erfindung wurde dargestellt, daß bei Erlöschen des Plasmabogens aufgrund des dadurch bedingten Wegfalls der durch ihn hervorgerufenen Bedämpfung der Resonanzschaltung die Spannung an der Elektrode 4 gegenüber dem menschlichen Gewebe 1 in kürzestmöglicher Zeit wieder über die Zündspannung hinaus ansteigt. Für die Realisierung dieses Vorteils ist dabei keine Regeleinrichtung, wie z.B. die Regelung des Stromflußwinkels, notwendig.

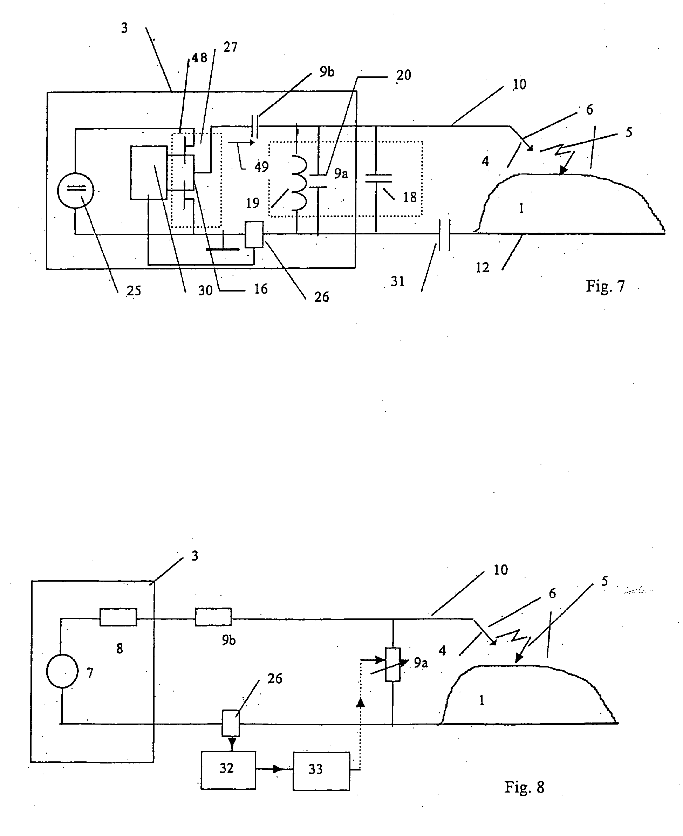

Gemäß Fig. 7 ist der Hochfrequenzgenerator 3 aus einer Gleichspannungsquelle 25 und einer Komplementärschaltung 48 aus Schalttransistoren 27 realisiert. Die Schalttransistoren 27 arbeiten im Gegentaktbetrieb und werden durch einen Pulsgeber 30 angesteuert. Beide Transistoren sind wechselseitig voll leitend bzw. voll sperrend und arbeiten im reinen Schaltbetrieb. Dadurch steht an ihrer gemeinsamen Quellelektrode 16 eine Rechteckspannung mit dem Mittelwert der halben Spannung der Gleichspannungsquelle 25 bereit. Diese Rechteckspannung wird unmittelbar der Resonanzschaltung 2, bestehend aus erstem Blindwiderstand 9a und zweitem Blindwiderstand 9b, zugeführt.

Mit Hilfe der Stromauskopplung 26 werden im Pulsgeber 30 Pulse abgeleitet, welche die Schalttransistoren 27 synchron ansteuern, so daß sich aus der Gesamtanordnung ein selbstschwingender Oszillator gestalten läßt. Aufgrund der frequenzselektiven Wirkung der Resonanzschaltung 2 läßt die Serienresonanzschaltung nur sinusförmige Ströme mit der Frequenz der Grundschwingung der Rechteckspannung zu. Bei richtiger Einstellung der Gesamtschaltung werden die Schalttransistoren 27 jeweils im zeitlichen Nulldurchgang des Resonanzkreisstroms 49 geschaltet. Eine solche Anordnung ist besonders verlustarm, weil die Transistoren im reinen Schaltbetrieb arbeiten und jeder Transistor nur dann Strom führt, wenn die an ihm liegende Spannung bis auf die Restspannung verschwindet.

Bei Verwendung eines Hochfrequenzgenerators 3 mit vorgegebener Eigenfrequenz muß die Resonanzschaltung 2, bestehend aus erstem Blindwiderstand 9a und zweitem Blindwiderstand 9b, in ihrer Resonanzfrequenz auf die Frequenz des Hochfrequenzgenerators 3 abgestimmt werden. Dies kann, wie in Fig. 8 dargestellt, durch Regelung des ersten Blindwiderstands 9a automatisch erfolgen, indem der Strom im Resonanzkreis mit Hilfe der Stromauskopplung 26 in einem Amplitudenindikator 32 ermittelt und mit Hilfe eines Reglers 33 die Resonanzkreisstromstärke 49 auf ein Maximum geregelt wird.

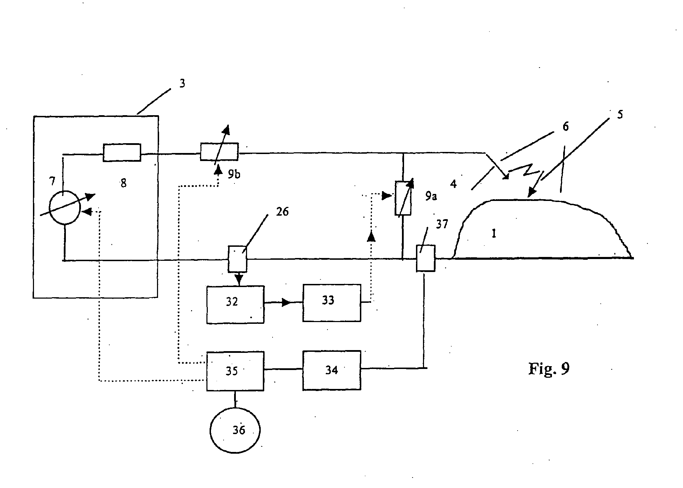

Fig. 9 zeigt in einer vorteilhaften Ausgestaltung der Erfindung, auf welche Weise auch die Intensität des Stroms im Plasmabogen 5 geregelt werden kann. Dies erfolgt mit Hilfe einer Patientenstromauskopplung 37 und eines Patientenstromindikators 34 in Verbindung mit einem Patientenstromregler 35, dem gleichzeitig ein Sollwert mit Hilfe eines Patientenstrom-Sollwertgebers 36 vorgegeben wird. Durch Einstellung des zweiten Blindwiderstands 9b und der Ausgangsspannung der Hochfrequenzspannungsquelle 7 wird der Plasmastrom auf den mit dem Sollwertgeber 36 vorgegebenen, gewünschten Wert eingestellt.

Eine besonders einfach realisierbare Einstellung der gewünschten Intensität des Stroms in Plasmabogen 5 kann - wie in den Figuren 5a und 6a gezeigt - auch durch Wahl eines Festkondensators als zweiter Blindwiderstand 9b erfolgen, und die Regelung kann trägheitslos durch Einstellung der Ausgangsspannung der Hochfrequenzspannungsquelle 7 durchgeführt werden. Die trägheitslose Regelung der Hochfrequenzspannungsquelle kann z.B. in der Anordnung in Fig. 7 dadurch erfolgen, daß die Gleichspannungsquelle 25 auf an sich bekannte Weise trägheitslos in ihrer Spannung geregelt wird und somit die hochfrequente Rechteckspannung an den Quellelektroden 16 geregelt und in der Folge davon die Intensität des Stroms in Plasmabogen 5 trägheitslos geregelt wird.

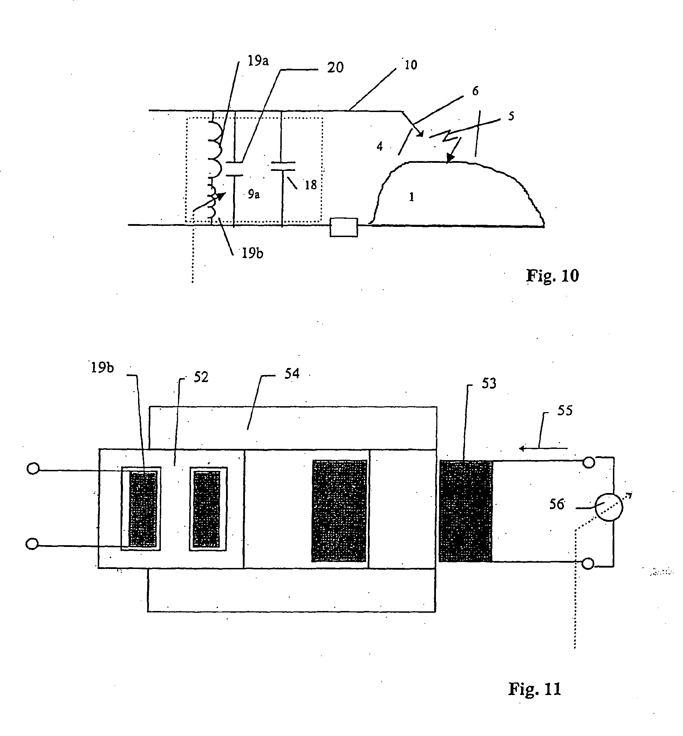

Hierbei erweist es sich als besonders vorteilhaft, auch die Einstellung der Resonanzfrequenz der Resonanzschaltung 2 auf die Frequenz des Hochfrequenzgenerators 3 trägheitslos zu gestalten. Dies kann auf vorteilhafte Weise z.B. - wie in Fig. 10 - durch Regelung des ersten Blindwiderstands 9a erfolgen, welcher in eine invariable Teilinduktivität 19a und eine variable Induktivität 19b zum Zwecke der Abstimmung unterteilt ist. Diese variable Induktivität 19b zur trägheitslosen Abstimmung der Resonanz kann ebenso für Anordnungen gemäß den Figuren 8 und 9 eingesetzt werden. Die Variation der Induktivität 19b kann, wie in Fig. 11 dargestellt, z.B. durch Vormagnetisierung eines Ferritkerns mit variabler Permeabilität 52 erfolgen. Die Vormagnetisierung wird mit Hilfe des Vormagnetisierungsjochs 54 durch die vom Vormagnetisierungsstrom 55 duchflossene Vormagnetisierungswicklung 53 verändert. Der variable Vormagnetisierungsstrom 55 wird dabei mit Hilfe der vom Regler 33 gesteuerten, auf an sich bekannte Weise gestalten, variablen Gleichspannungsquelle 56 eingestellt. Ein auf diese Weise gestaltetes Blindelement zur Gestaltung eines ersten Blindwiderstands 9a oder ggfs. auch eines zweiten Blindwiderstands 9b besitzt den Vorzug, eine hohe Blindenergie zu speichern, ohne Verzerrungen der Hochfrequenzschwingung hervorzurufen.

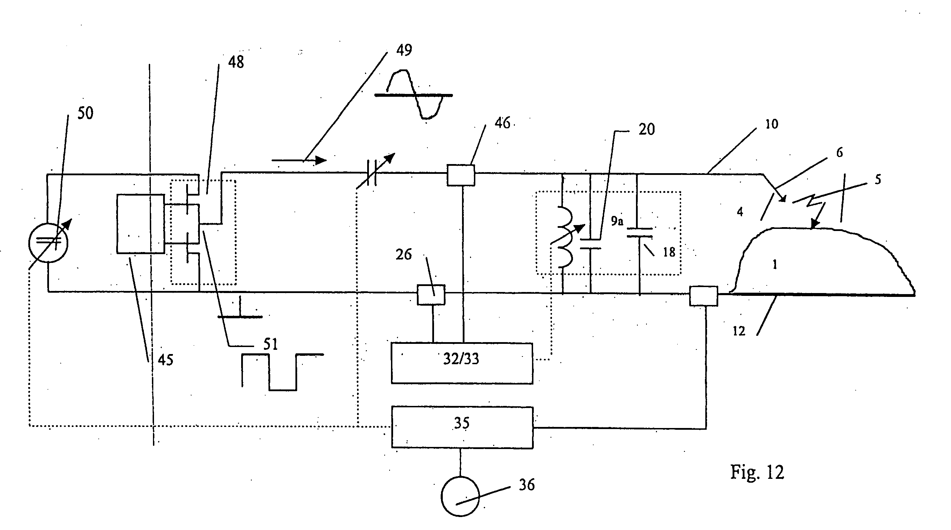

In Fig. 12 ist eine Anordnung mit Abstimmung und Einstellung der Intensität des Plasmastroms gezeigt, wie in Fig. 9 erläutert. Der Hochfrequenzgenerator ist, ähnlich wie in Fig. 7 erläutert, mit Schalttransistoren 27 realisiert. Die Ansteuerung der Schalttransistoren 27 erfolgt jedoch in diesem Beispiel vorteilhaft mit einem selbsterregten Oszillator 45. Damit können Anschwingprobleme des Hochfrequenzgenerators 3 vermieden werden. Die Intensität des Plasmastroms kann durch Variation der Spannung eines regelbaren Netzteils 50, durch Umschalten des Kondensators im zweiten Blindwiderstand 9b oder durch beide Maßnahmen gesteuert werden. Eine trägheitslose Regelung der Resonanz erfolgt vorteilhaft durch Variation der Induktivität im ersten Blindwiderstand 9a durch Vormagnetisierung des magnetischen Kerns der Induktivität.

### BEZUGSZEICHENLISTE

- 1: menschliches Gewebe
- 2: Resonanzschaltung
- 3: Hochfrequenzgenerator
- 4: Elektrode
- 5: Plasmabogen
- 6: Plasmastrecke
- 7: Hochfrequenzspannungsquelle
- 8: Innenwiderstand
- 9a: erster Blindwiderstand
- 9b: zweiter Blindwiderstand
- 10: Zuleitung
- 11: Masseanschluß
- 12: äußere HF-Masseelektrode
- 13: koaxialer Schirmleiter
- 14: innere HF-Masseelektrode
- 16: Quellelektroden
- 17: Vor- bzw. Rückströme
- 18: wirksame Kapazität
- 19: Parallelinduktivität
- 19a: Teilinduktivität
- 19b: variable Induktivität
- 20: Parallelkondensator
- 21: Serieninduktivität
- 22: Serienkapazität
- 23: Isolationsschicht
- 24: Edelgasstrom
- 25: Gleichspannungsquelle
- 26: Stromauskopplung
- 27: Schalttransistoren
- 28: geeigneter Kondensator
- 29: Transformator
- 30: Pulsgeber
- 31: galvanischer Trennkondensator
- 32: Amplitudenindikator
- 33: Regler
- 34: Patientenstromindikator
- 35: Patientenstromregler
- 36: Patientenstrom-Sollwertgeber
- 37: Patientenstromauskopplung
- 38: isolierender Schlauch
- 39: erste Kondensatorbatterie
- 40: zweite Kondensatorbatterie
- 41: erster Schalter
- 42: zweiter Schalter
- 43: Batterie von Induktivitäten
- 44: vorgegebener HF-Generator
- 45: selbsterregter Oszillator
- 46: Spannungsauskopplung
- 47: Resonanztransformationsschaltung
- 48: geschaltete Transistorendstufe
- 49: Resonanzkreisstrom
- 50: regelbares Netzteil
- 51: rechteckförmige HF-Spannung
- 52: Ferritkern mit variabler Permeabilität
- 53: Vormagnetisierungswicklung
- 54: Vormagnetisierungsjoch
- 55: Vormagnetisierungsstrom
- 56: variable Gleichspannungsquelle

## Patentansprüche

1. Hochfrequenzeinrichtung zur Erzeugung eines Plasmabogens (5) für die Behandlung biologischen Gewebes (1), insbesondere in der Medizin, mit einem Hochfrequenzgenerator (3) mit einem Innenwiderstand (8) und einer an diesen über eine flexible Zuleitung (10) angeschlossenen Elektrode (4) zur Erzeugung des Plasmabogens (5) in einer Plasmastrecke (6) zwischen der Elektrode (4) und dem ebenfalls mit dem Hochfrequenzgenerator (3) elektrisch verbundenen biologischen Gewebe (1), wobei
im Hochfrequenzgenerator (3) eine Hochfrequenzspannungsquelle (7) vorhanden ist, an die eine Resonanzschaltung (2), umfassend eine Serienschaltung eines kapazitiv wirkenden ersten Blindwiderstandes (9a) und eines induktiv wirkenden zweiten Blindwiderstandes (9b), mit einer Resonanzfrequenz auf der Frequenz der von der Hochfrequenzspannungsquelle (7) abgegebenen Hochfrequenzschwingung angeschlossen ist, wobei die Spannung zur Erzeugung des Plasmabogens (5) von dem ersten oder zweiten Blindwiderstand (9a, 9b) abgeleitet ist,
wobei der erste und zweite Blindwiderstand (9a, 9b) eine im wesentlichen gleiche Größe bei der Resonanzfrequenz aufweisen und die Plasmastrecke (6) mindestens abschnittsweise parallel zu dem ersten (9a) der beiden Blindwiderstände geschaltet ist,
**dadurch gekennzeichnet, daß**
die Größe der Blindwiderstände (9a, 9b) in Abstimmung auf die Spannungsamplitude der Hochfrequenzspannungsquelle (7) derart gewählt ist, daß einerseits im Zustand des erloschenen Plasmas die Hochfrequenzspannung an der Plasmastrecke (6) infolge der Resonanz mindestens gleich der zur Zündung des Plasmabogens (5) notwendigen Spannung und andererseits im Zustand des bestehenden Plasmabogens (5) die Resonanz durch den Leitwert der Plasmastrecke (6) bedämpft ist und die Größe der Blindwiderstände in Verbindung mit deren Verlusten eine für die medizinische Anwendung geeignete Hochfrequenzstromstärke in der Plasmastrecke (6) bestimmt.

2. Hochfrequenzeinrichtung zur Erzeugung eines Plasmabogens (5) gemäß Anspruch 1,
**dadurch gekennzeichnet, daß**
der erste Blindwiderstand (9a) der Resonanzschaltung (2), von welchem die Spannung zur Erzeugung des Plasmabogens (5) abgeleitet wird, aus der Kapazität mindestens eines Kondensators (20) und der Kapazität (18), welche zwischen der Zuleitung (10) und der Elektrode (4) einerseits sowie dem über eine Masseelektrode (12) mit der Resonanzschaltung (2) verbundenen Gewebe (1) sowie einer Zuleitung von der Resonanzschaltung (2) zu dieser Masseelektrode (12) andererseits vorhanden ist, gebildet ist.

3. Hochfrequenzeinrichtung zur Erzeugung eines Plasmabogens (5) nach Anspruch 1 oder 2,
**dadurch gekennzeichnet, daß**
der Stromwinkel des Generatorstroms im wesentlichen konstant ist.

4. Hochfrequenzeinrichtung zur Erzeugung eines Plasmabogens (5) nach einem der Ansprüche 1 bis 3,
**dadurch gekennzeichnet, daß**
der Innenwiderstand (8) der Hochfrequenzspannungsquelle (7) wesentlich kleiner ist als der Blindwiderstandswert des ersten Blindwiderstands (9a) bzw. des zweiten Blindwiderstands (9b) bei der Resonanzfrequenz des Resonanzkreises (2).

5. Hochfrequenzeinrichtung zur Erzeugung eines Plasmabogens (5) nach einem der vorangehenden Ansprüche,
**dadurch gekennzeichnet, daß**
das menschliche Gewebe (1) mit einem das Massepotential führenden Anschluß des Hochfrequenzgenerators (3) verbunden ist,
der erste Blindwiderstand (9a) der Resonanzschaltung (2) aus einer Parallelschaltung eines Parallelkondensators (20) mit der Zuleitung (10) mit daran angeschlossener Elektrode (4) gebildet ist und
eine aus der Zuleitung (10) und der Elektrode (4) gebildete gemeinsame wirksame Kapazität (18) zusammen mit dem Parallelkondensator (20) einen zu dem als Serieninduktivität (21) realisierten zweiten Blindwiderstand (9b) negativ gleich großen Blindwiderstand bildet.

6. Hochfrequenzeinrichtung zur Erzeugung eines Plasmabogens (5) nach Anspruch 5,
**dadurch gekennzeichnet, daß**
der zweite Blindwiderstand (9b) als induktiver Blindwiderstand, bestehend aus einer Parallelschaltung aus einer Induktivität (21) und einer Kapazität (22), realisiert ist und die aus der Zuleitung (10) und Elektrode (4) gebildete gemeinsame wirksame Kapazität (18) zusammen mit dem Kondensator (20) einen zu dem durch die Parallelschaltung aus der Induktivität (21) und Kapazität (22) realisierten zweiten Blindwiderstand (9b) negativ gleich großen Blindwiderstand bildet.

7. Hochfrequenzeinrichtung zur Erzeugung eines Plasmabogens (5) nach einem der Ansprüche 1 bis 6,
**dadurch gekennzeichnet, daß**
der erste Blindwiderstand (9a) der Resonanzschaltung (2) aus der Parallelschaltung einer Induktivität (19) mit der Zuleitung (10) mit daran angeschlossener Elektrode (4) gebildet ist und die aus der Zuleitung (10) und der Elektrode (4) gebildete gemeinsam wirkende Kapazität (18) zusammen mit der Induktivität (19) einen zu dem als Serienkapazität (22) realisierten zweiten Blindwiderstand (9b) negativ gleich großen Blindwiderstand bildet.

8. Hochfrequenzeinrichtung zur Erzeugung eines Plasmabogens (5) nach Anspruch 7,
**dadurch gekennzeichnet, daß**
das Gewebe (1) mit dem das Massepotential führenden Anschluß des Hochfrequenzgenerators (3) über eine an den Patienten angelegte Masseelektrode (12) verbunden ist.

9. Hochfrequenzeinrichtung zur Erzeugung eines Plasmabogens (5) nach einem der vorangehenden Ansprüche,
**dadurch gekennzeichnet, daß**
die Zuleitung (10) als isolierte unipolare Leitung zum Patienten und zur Elektrode (4) an der Operationsstelle ausgeführt ist.

10. Hochfrequenzeinrichtung zur Erzeugung eines Plasmabogens (5) nach Anspruche 8,
**dadurch gekennzeichnet, daß**
die Zuleitung (10) als Innenleiter einer koaxialen Leitung mit koaxialem Schirmleiter (13) mit einer gegen Kontakt mit dem Gewebe sichernden äußeren Isolationsschicht (23) zum Patienten und zur Elektrode (4) an der Operationsstelle ausgeführt und der koaxiale Schirmleiter (13) mit der an den Patienten angelegten Masseelektrode (12) verbunden ist.

11. Hochfrequenzeinrichtung zur Erzeugung eines Plasmabogens (5) nach einem der vorangehenden Ansprüche,
**dadurch gekennzeichnet, daß**
die Zuleitung (10) als Innenleiter einer koaxialen Leitung mit koaxialem Schirmleiter (13) mit einer gegen Kontakt mit dem Gewebe sichernden äußeren Isolationsschicht (23) zum Patienten und zur Elektrode (4) an der Operationsstelle ausgeführt ist und der koaxiale Schirmleiter (13) an seinem einen Ende mit dem das Massepotential führenden Anschluß des Hochfrequenzgenerators (3) leitend verbunden ist und an seinem anderen Ende in unmittelbarer Nähe der Operationsstelle eine mit dem Schirmleiter (13) leitend verbundene Masseelektrode (14) aufweist, welche durch flächige Berührung mit dem Gewebe (1) mit diesem elektrisch verbindbar ist.

12. Hochfrequenzeinrichtung zur Erzeugung eines Plasmabogens (5) nach einem der vorangehenden Ansprüche,
**dadurch gekennzeichnet, daß**
zur Erzielung einer hohen örtlichen Selektivität des Plasmabogens (5) ein Edelgasstrom (24), vorzugsweise ein Argongasstrom, mit niedriger Ionisationsfeldstärke auf das zu behandelnde Gewebe gerichtet ist, wobei das Edelgas im Innenleiter, innerhalb des koaxialen Schirmleiters oder in einer zu diesem parallelen Röhre zum Operationsort geführt ist.

13. Hochfrequenzeinrichtung zur Erzeugung eines Plasmabogens (5) nach einem der Ansprüche 6 bis 12,
**dadurch gekennzeichnet, daß**
zur Wahl verschiedener Stromstärken im Plasmabogen (5) der als Kapazität (22) realisierte zweite Blindwiderstand (9b) aus einer ersten Kondensatorgruppe (39) mit Hilfe eines ersten Schalters (41) geeignet ausgewählt ist und zur Erfüllung der Resonanzbedingung der Parallelinduktivität (19) und der wirksamen Kapazität (18) der Zuleitung (10) mit daran angeschlossener Elektrode (4) ein geeigneter Kondensator (28) parallelgeschaltet ist, welcher mit Hilfe eines zweiten Schalters (42) aus einer zweiten Kondensatorgruppe (40) ausgewählt ist.

14. Hochfrequenzeinrichtung zur Erzeugung eines Plasmabogens (5) nach Anspruch 5 der einem der Ansprüche 7 bis 12,
**dadurch gekennzeichnet, daß**
zur Wahl verschiedener Stromstärken im Plasmabogen (5) der als Serieninduktivität (21) realisierte zweite Blindwiderstand (9b) aus einer Gruppe von Induktivitäten (43) mit Hilfe eines ersten Schalters (41) geeignet ausgewählt ist und zur Erfüllung der Resonanzbedingung der wirksamen Kapazität (18) der Zuleitung (10) mit daran angeschlossener Elektrode (4) ein geeigneter Kondensator (28) parallelgeschaltet ist, welcher mit Hilfe eines zweiten Schalters (42) aus einer Kondensatorgruppe (39) ausgewählt ist.

15. Hochfrequenzeinrichtung zur Erzeugung eines Plasmabogens (5) nach einem der vorangehenden Ansprüche,
**dadurch gekennzeichnet, daß**
einem Hochfrequenzgenerator (44), welcher auch für andere Applikationen bestimmt ist, ein Transformator (29) mit mehreren wählbaren Einstellungen des Übersetzungsverhältnisses zugeordnet ist und mit diesem ein erster Schalter (41) zur Vorwahl des Übersetzungsverhältnisses verbunden ist.

16. Hochfrequenzeinrichtung zur Erzeugung eines Plasmabogens (5) nach einem der vorangehenden Ansprüche,
**gekennzeichnet durch**
eine hochohmige HF-Stromquelle (15) und eine Resonanztransformationsschaltung (47), die die hochohmige HF-Stromquelle in eine Hochfrequenzspannungsquelle (7) mit niederohmigem Innenwiderstand (8) umwandelt.

17. Hochfrequenzeinrichtung zur Erzeugung eines Plasmabogens (5) nach einem der vorangehenden Ansprüche,
**dadurch gekennzeichnet, daß**
der Hochfrequenzgenerator (3) durch eine Hochfrequenzspannungsquelle (7) mit periodischer Signalform, vorzugsweise einer Rechteckform, mit niedrigem Innenwiderstand (8) gebildet ist, welcher insbesondere in der Größenordnung der Wellenwiderstände von Koaxialleitungen liegt.

18. Hochfrequenzeinrichtung zur Erzeugung eines Plasmabogens (5) nach Anspruch 17,
**dadurch gekennzeichnet, daß**
der Hochfrequenzgenerator (3) mit Rechteck-Signalform durch eine Gleichspannungsquelle (25) mit einer hierzu parallelgeschalteten Komplementärschaltung aus im Gegentakt betriebenen Schalttransistoren (27) gebildet ist und diese von einem Pulsgeber (30) angesteuert sind, welcher mit Hilfe einer Stromauskopplung (26) zur Messung des Resonanzkreisstromes synchronisiert ist, und die Ausgangsspannung des derart gebildeten Hochfrequenzgenerators (3) an der gemeinsamen Quellelektrode (16) der Schalttransistoren (27) vorliegt.

19. Hochfrequenzeinrichtung zur Erzeugung eines Plasmabogens (5) nach einem der vorangehenden Ansprüche,
**dadurch gekennzeichnet, daß**
der Hochfrequenzgenerator (3) eine vorgegebene Eigenfrequenz besitzt und eine Regeleinrichtung vorhanden ist, durch die die Resonanz in der Resonanzschaltung (2) über Einstellung mindestens eines der beiden Blindwiderstände regelbar ist.

20. Hochfrequenzeinrichtung zur Erzeugung eines Plasmabogens (5) nach Anspruch 19,
**dadurch gekennzeichnet, daß**
die Resonanz in der Resonanzschaltung (2) durch Einstellung des ersten Blindwiderstands (9a) dadurch gegeben ist, daß eine Stromauskopplung (26) im Stromkreis der Resonanzschaltung (2) vorhanden ist und der ausgekoppelte Strom einem Amplitudenindikator (32) zugeführt und in diesem ausgewertet wird und dem Amplitudenindikator ein Regler (33) zur Einstellung des Blindwiderstands (9a) nachgeschaltet ist, in welchem das Signal des Amplitudenindikators (32) derart ausgewertet ist, daß der Resonanzstrom und damit die Spannung am ersten Blindwiderstand (9a) auf ein Maximum eingestellt wird.

21. Hochfrequenzeinrichtung zur Erzeugung eines Plasmabogens (5) nach Anspruch 19 oder 20,
**dadurch gekennzeichnet, daß**
die Regeleinrichtung einen Sollwertgeber (36) zur Voreinstellung des Stroms in Plasmabogen (5) und damit des Patientenstroms aufweist.

22. Hochfrequenzeinrichtung zur Erzeugung eines Plasmabogens (5) nach Anspruch 21.
**dadurch gekennzeichnet, daß**
eine Patientenstromauskopplung (37), ein Patientenstromindikator (34) und ein Patientenstromregler (35) vorgesehen sind und der Strom im Plasmabogen (5) durch Einstellung des zweiten Blindwiderstands (9b) und der Hochfrequenzspannungsquelle (7) auf den mit dem Sollwertgeber (36) vorgegebenen Wert regelbar ist.

23. Hochfrequenzeinrichtung zur Erzeugung eines Plasmabogens (5) nach einem der vorangehenden Ansprüche,
**dadurch gekennzeichnet, daß**
mindestens einer der beiden Blindwiderstände (9a, 9b) ganz oder teilweise durch eine variable Induktivität (19b) gebildet ist, deren Induktivitätswert trägheitslos durch Vormagnetisierung eines Ferritkerns mit variabler Permeabilität (52) gegeben ist, wobei die Vormagnetisierung mit Hilfe einer von einem Vormagnetisierungsstrom (55) durchflossenen Vormagnetisierungswicklung (53) eingestellt ist.

24. Hochfrequenzeinrichtung zur Erzeugung eines Plasmabogens (5) nach Anspruch 23,
**dadurch gekennzeichnet, daß**
der variable Vormagnetisierungsstrom (55) mit Hilfe einer gesteuerte variablen Gleichspannungsquelle (56) eingestellt ist.

25. Hochfrequenzeinrichtung zur Erzeugung eines Plasmabogens (5) nach einem der vorangehenden Ansprüche,
**dadurch gekennzeichnet, daß**
die Hochfrequenzspannungsquelle (7) und mindestens Teile der Resonanzschaltung (2), insbesondere zusammen mit Regel- bzw. Einstelleinrichtungen zur Einstellung der Resonanzfrequenz bzw. zur Einstellung der Intensität des Plasmastroms, in einem die Sicherheit des Patienten gewährleistenden Gehäuse untergebracht sind, an welches die Zuleitung (10) mit daran angeschlossener Elektrode (4) angeschlossen ist.

## Claims

1. High-frequency device for generating a plasma arc (5) for the treatment of biological tissue (1), in particular in medicine, with a high-frequency generator (3) having an internal resistor (8) and an electrode (4) connected thereto by means of a flexible lead (10) for generating the plasma arc (5) in a plasma gap (6) between the electrode (4) and the biological tissue (1), which is also electrically connected to the high-frequency generator (3),
wherein a high-frequency voltage source (7) is also provided in the high-frequency generator (3), to which source a resonance circuit (2), comprising a series connection of a capacitively acting first reactance (9a) and an inductively acting second reactance (9b), having a resonance frequency at the frequency of the high-frequency oscillation emitted by the high-frequency voltage source (7),
wherein the voltage for generating the plasma arc (5) is derived from the first or the second reactance (9a, 9b),
wherein the first and second reactances (9a, 9b) are substantially of the same magnitude at the resonance frequency and the plasma gap (6) is connected at least in sections to the first (9a) of the two reactances,
**characterised in that**
the magnitude of the reactances (9a, 9b) is matched to the voltage amplitude of the high-frequency voltage source (7) in such a way that, on the one hand, in a state wherein the plasma arc is extinguished, the high-frequency voltage at the plasma gap (6) is, as a result of the resonance, at least equal to the voltage required to ignite the plasma arc (5) and, on the other hand, in a state where the plasma arc (5) exists the resonance is attenuated by the conductance of the plasma gap (6) and the magnitude of the reactances, in conjunction with their losses, determines a high-frequency current intensity in the plasma gap (6) that is suited to medical application.

2. High-frequency device for generating a plasma arc (5) according to Claim 1
**characterised in that**
the first reactance (9a) of the resonance circuit (2) from which the voltage for generating the plasma arc (5) is derived is formed from the capacitance of at least one capacitor (20) and the capacitance (18) that is provided between the lead (10) and the electrode (4) on the one hand and between the tissue (1) connected to the resonance circuit (2) by means of an earth electrode (12) and a lead from the resonance circuit (2) to this earth electrode (12) on the other.

3. High-frequency device for generating a plasma arc (5) according to Claim 1 or 2, **characterised in that** the angle of current of the generator current is substantially constant.

4. High-frequency device for generating a plasma arc (5) according to one of the Claims 1 to 3,
**characterised in that**
the internal resistance (8) of the high-frequency voltage source (7) is substantially smaller than the reactance value of the first reactance (9a) or of the second reactance (9b) at the resonance frequency of the resonance circuit (2).

5. High-frequency device for generating a plasma arc (5) according to one of the preceding claims,
**characterised in that**
the human tissue (1) is connected to a connection of the high-frequency generator (3) carrying earth potential,
the first reactance (9a) of the resonance circuit (2) is formed from a parallel connection of a parallel capacitor (20) to the lead (10) with connected electrode (4) and
a common effective capacitance (18) formed from the lead (10) and the electrode (4) together with the parallel capacitor (20) forms a reactance of a negatively equivalent value to that of the second reactance (9b) realised as a series inductor (21).

6. High-frequency device for generating a plasma arc (5) according to Claim 5,
**characterised in that**
the second reactance (9b) is realised as an inductive resistance, consisting of a parallel connection of an inductor (21) and a capacitance (22), and the common effective capacitance (18) formed from the lead (10) and the electrode (4) together with the capacitor (20) forms a reactance of a negatively equivalent value to that of the second reactance (9b) realised by the parallel connection consisting of the inductor (21) and capacitance (22).

7. High-frequency device for generating a plasma arc (5) according to one of the Claims 1 to 6,
**characterised in that**
the first reactance (9a) of the resonance circuit (2) is formed from the parallel connection of an inductor (19) to the lead (10) with connected electrode (4) and the common effective capacitance (18) formed from the lead (10) and the electrode (4) together with the inductor (19) forms a reactance of a negatively equivalent value to that of the second reactance (9b) realised as a series capacitance (22).

8. High-frequency device for generating a plasma arc (5) according to Claim 7,
**characterised in that**
the tissue (1) is connected to the connection of the high-frequency generator (3) carrying earth potential by means of an earth electrode (12) applied to the patient.

9. High-frequency device for generating a plasma arc (5) according to one of the preceding claims,
**characterised in that**
the lead (10) is configured as an insulated unipolar lead to the patient and the electrode (4) on the operation site.

10. High-frequency device for generating a plasma arc (5) according to Claim 8,
**characterised in that**
the lead (10) is configured as the inner conductor of a coaxial lead to the patient and the electrode (4) on the operation site that has a coaxial shield conductor (13) with an outer insulating layer (23) guarding against contact with the tissue and the coaxial shield conductor (13) is connected to the earth electrode (12) applied to the patient.

11. High-frequency device for generating a plasma arc (5) according to one of the preceding claims,
**characterised in that**
the lead (10) is configured as the inner conductor of a coaxial lead to the patient and the electrode (4) on the operation site that has a coaxial shield conductor (13) with an outer insulating layer (23) guarding against contact with the tissue and the coaxial shield conductor (13) is connected conductively at one end with the connection of the high-frequency generator (3) carrying earth potential and has at its other end in immediate proximity to the operation site an earth electrode (14) conductively connected to the shield conductor (13), which electrode can be electrically connected to the tissue (1) through surface-area contact therewith.

12. High-frequency device for generating a plasma arc (5) according to one of the preceding claims,
**characterised in that**,
in order to achieve high local selectivity of the plasma arc (5), a stream of inert gas (24), preferably a stream of argon gas, with low ionisation field strength is directed at the tissue being treated, wherein the inert gas is guided to the operation site in the inner conductor, inside the coaxial shield conductor or in a parallel tube.

13. High-frequency device for generating a plasma arc (5) according to one of the Claims 6 to 12,
**characterised in that**,
in order to select different current intensities in the plasma arc (5), the second reactance (9b) realised as a capacitance (22) is selected from a first group of capacitors (39) by means of a first switch (41) and, in order to satisfy the resonance condition of the parallel inductor (19) and the effective capacitance (18) of the lead (10) with connected electrode (4), a suitable capacitor (28) is connected in parallel, which is selected from a second group of capacitors (40) by means of a second switch (42).

14. High-frequency device for generating a plasma arc (5) according to Claim 5 or one of the Claims 7 to 12,
**characterised in that**,
in order to select different current intensities in the plasma arc (5), the second reactance (9b) realised as a series inductor (21) is selected from a group of inductors (43) by means of a first switch (41) and, in order to satisfy the resonance condition of the effective capacitance (18) of the lead (10) with connected electrode (4), a suitable capacitor (28) is connected in parallel, which is selected from a group of capacitors (39) by means of a second switch (42).

15. High-frequency device for generating a plasma arc (5) according to one of the preceding claims,
**characterised in that**
a transformer (29) with several selectable settings of the transformation ratio is assigned to a high-frequency generator (44), which is also intended for other applications, and a first switch (41) for preselecting the transformation ratio is connected to this transformer.

16. High-frequency device for generating a plasma arc (5) according to one of the preceding claims,
**characterised by**
a high-impedance HF-current source (15) and a resonance transformation circuit (47), which converts the high-impedance HF-current source into a high-frequency voltage source (7) with a low-impedance internal resistance (8).

17. High-frequency device for generating a plasma arc (5) according to one of the preceding claims,
**characterised in that**
the high-frequency generator (3) is formed by a high-frequency voltage source (7) with a periodic signal shape, preferably a rectangular shape, with a low internal resistance (8), which in particular is in the order of magnitude of the characteristic wave impedances of coaxial leads.

18. High-frequency device for generating a plasma arc (5) according to Claim 17,
**characterised in that**
the high-frequency generator (3) with a rectangular signal shape is formed by a direct voltage source (25), with a complementary circuit connected in parallel to this, from switching transistors (27) operated in push-pull mode and these are actuated by a pulse generator (30), which is synchronised to measure the resonance circuit current by means of a current decoupler (26), and the output voltage of the high-frequency generator (3) formed in such a way is applied to the common source electrode (16) of the switching transistors (27).

19. High-frequency device for generating a plasma arc (5) according to one of the preceding claims,
**characterised in that**
the high-frequency generator (3) has a predefined natural frequency and a controlling device is provided by means of which the resonance in the resonance circuit (2) can be controlled by adjusting at least one of the two reactances.

20. High-frequency device for generating a plasma arc (5) according to Claim 19,
**characterised in that**
the resonance in the resonance circuit (2) produced by adjusting the first reactance (9a) is determined by the fact that a current decoupler (26) is provided in the circuitry of the resonance circuit (2) and the decoupled current is conducted to an amplitude indicator (32) and is evaluated there, and downstream from the amplitude indicator is a controller (33) for setting the reactance (9a), in which controller the signal from the amplitude indicator (32) is evaluated in such a way that the resonance current and thus the voltage on the first reactance (9a) is set to a maximum.

21. High-frequency device for generating a plasma arc (5) according to Claim 19 or 20,
**characterised in that**
the controlling device comprises a setpoint generator (36) for presetting the current in the plasma arc (5) and thus the patient current.

22. High-frequency device for generating a plasma arc (5) according to Claim 21,
**characterised in that**
a patient current decoupler (37), a patient current indicator (34) and a patient current controller (35) are provided and the current in the plasma arc (5) can be controlled by setting the second reactance (9b) and the high-frequency voltage source (7) to the value predefined by the setpoint generator (36).

23. High-frequency device for generating a plasma arc (5) according to one of the preceding claims,
**characterised in that**
at least one of the two reactances (9a, 9b) is wholly or partially formed by a variable inductor (19b), the inductance value of which is provided in an inertia free manner by premagnetising a ferrite core with variable permeability (52), wherein the magnetic bias is set by means of a bias winding (53) through which a magnetic biasing current (55) flows.

24. High-frequency device for generating a plasma arc (5) according to Claim 23,
**characterised in that**
the variable magnetic biasing current (55) is set by means of a controlled variable direct voltage source (56).

25. High-frequency device for generating a plasma arc (5) according to one of the preceding claims,
**characterised in that**
the high-frequency voltage source (7) and at least parts of the resonance circuit (2), in particular together with controlling or setting devices for setting the resonance frequency or setting the intensity of the plasma current, are accommodated in a housing guaranteeing the safety of the patient, to which the lead (10) with connected electrode (4) is attached.

## Revendications

1. Dispositif haute fréquence, permettant de produire un arc de plasma (5) destiné au traitement de tissus biologiques (1), en particulier en médecine, comprenant un générateur haute fréquence (3) comportant une résistance interne (8) et une électrode (4) raccordée à celle-ci par un câble d'alimentation flexible (10) pour produire l'arc de plasma (5) dans un parcours de plasma (6) entre l'électrode (4) et également le tissu biologique (1) relié électriquement au générateur haute fréquence (3),
une source de tension haute fréquence (7) étant présente dans le générateur haute pression (3), à laquelle un montage en résonance (2) comprenant un commutateur en série d'une première réactance capacitive (9a) et d'une deuxième résistance inductive (9b) est raccordé avec une fréquence de résonance, à la fréquence de l'oscillation haute fréquence indiquée par la source de tension haute fréquence (7), la tension de production d'un arc de plasma (5) étant dérivée de la première ou de la deuxième réactance (9a, 9b),
**caractérisé en ce que** la première et la deuxième réactances (9a, 9b) présentent des ampleurs essentiellement identiques à la fréquence de résonance et le parcours du plasma (6) est commuté partiellement en parallèle à la première (9a) des deux réactances, l'ampleur de la réactance (9a, 9b) étant choisie en fonction de l'amplitude de tension de la source de tension haute fréquence (7) de telle sorte que d'une part, à l'état de plasma éteint, la tension de haute fréquence au niveau du parcours du plasma (6) consécutive à la résonance, est au moins égale à la tension nécessaire pour l'allumage de l'arc de plasma (5) et d'autre part, la résonance est atténuée par la conductance du parcours de plasma (6) et l'ampleur des réactances en tenant compte de leurs pertes, détermine une intensité de courant haute fréquence appropriée dans le parcours de plasma (6) pour l'application médicale.

2. Dispositif haute fréquence, permettant de produire un arc de plasma (5) selon la revendication 1,
**caractérisé en ce que** la première réactance (9a) du montage en résonance (2) duquel la tension permettant de produire un arc de plasma (5) provient, est formée à partir de la capacité (18) d'au moins un condensateur (20) et de la capacité (18) qui est présente entre le câble d'alimentation (10) et l'électrode (4) d'une part, et du tissu (1) relié par le biais d'une électrode de masse (12) avec le montage en résonance (2) et d'une câble d'alimentation du montage en résonance (2) à cette électrode de masse (12) d'autre part.

3. Dispositif haute fréquence, permettant de produire un arc de plasma (5) selon la revendication 1 ou 2,
**caractérisé en ce que** l'angle de courant du courant du générateur est essentiellement constant.

4. Dispositif haute fréquence, permettant de produire un arc de plasma (5) selon l'une des revendications 1 à 3,
**caractérisé en ce que** la résistance interne (8) de la source de tension haute fréquence (7) est essentiellement plus faible que la valeur de réactance de la première réactance (9a) ou de la deuxième réactance (9b) pour la fréquence de résonance du circuit de résonance (2).

5. Dispositif haute fréquence, permettant de produire un arc de plasma (5) selon l'une des revendications précédentes,
**caractérisé en ce que** le tissu humain (1) est relié à un raccordement conduisant le potentiel de masse du générateur haute fréquence (3), la première réactance (9a) du montage en résonance (2) est formée à partir d'un montage parallèle d'un condensateur parallèle (20), le câble d'alimentation (10) étant raccordé à l'électrode (4), et
une capacité efficace commune formée par le câble d'alimentation (10) et l'électrode (4), conjointement avec le condensateur parallèle (20) forme une réactance négative de même ampleur par rapport à la deuxième réactance (9b) réalisée en tant qu'inductance de série (21).

6. Dispositif haute fréquence, permettant de produire un arc de plasma (5) selon la revendication 5,
**caractérisé en ce que** la deuxième réactance (9b) est réalisée en tant que réactance inductive constitué d'un montage parallèle d'une inductance (21) et d'une capacité (22) et la capacité (18) efficace commune formée par le câble d'alimentation (10) et l'électrode (4) conjointement avec le condensateur (20) forme une réactance négative de même ampleur par rapport à la deuxième réactance (9b) réalisée par le montage parallèle de l'inductance (21) et de la capacité (22).

7. Dispositif haute fréquence, permettant de produire un arc de plasma (5) selon l'une des revendications 1 à 6,
**caractérisé en ce que** la première réactance (9a) du montage en résonance (2) est formée du montage parallèle d'une inductance (19), le câble d'alimentation étant raccordé à l'électrode (4) et la capacité (18) efficace commune formée à partir du câble d'alimentation (10) et de l'électrode (4) conjointement avec l'inductance (19) forme une réactance négative de même ampleur par rapport à la deuxième réactance (9b) réalisée en tant que capacité de série (22).

8. Dispositif haute fréquence, permettant de produire un arc de plasma (5) selon la revendication 7,
**caractérisé en ce que** le tissu (1) est relié au raccordement du générateur haute fréquence (3) conduisant le potentiel de masse par une électrode de masse (12) placée sur le patient.

9. Dispositif haute fréquence, permettant de produire un arc de plasma (5) selon l'une des revendications précédentes,
**caractérisé en ce que** le câble d'alimentation (10) est configuré en tant que câble unipolaire isolé par rapport au patient et à l'électrode (4) au site d'opération.

10. Dispositif haute fréquence, permettant de produire un arc de plasma (5) selon la revendication 8,
**caractérisé en ce que** le câble d'alimentation (10) est configuré en tant que conducteur central d'un câble d'alimentation coaxial avec un conducteur écran coaxial (13) présentant une couche isolante (23) externe contre un contact étroit avec le tissu, par rapport au patient et à l'électrode (4) au site d'opération et le conducteur écran coaxial (13) est relié à l'électrode de masse placée sur le patient.

11. Dispositif haute fréquence, permettant de produire un arc de plasma (5) selon l'une des revendications précédentes,
**caractérisé en ce que** le câble d'alimentation (10) est configuré en tant que conducteur central d'un câble d'alimentation coaxial avec un conducteur écran coaxial (13) présentant une couche isolante (23) externe contre un contact étroit avec le tissu, par rapport au patient et à l'électrode (4) au site d'opération et le conducteur écran coaxial (13) est relié à l'une de ses extrémités, avec le raccordement conduisant le potentiel de masse du générateur haute fréquence (3) relié conductivement et présente à son autre extrémité, à proximité immédiate du site d'opération, une électrode de masse (14) reliée conductivement avec le conducteur écran (13) qui peut être relié à celle-ci électriquement par contact superficiel avec le tissu (1).

12. Dispositif haute fréquence, permettant de produire un arc de plasma (5) selon l'une des revendications précédentes,
**caractérisé en ce que**, pour obtenir une sélectivité locale élevée de l'arc de plasma (5), un courant de gaz rare (24), de préférence, un courant de gaz d'argon, est dirigé avec une puissance de champ d'ionisation inférieure sur le tissu à traiter, le gaz rare étant dirigé dans le conducteur central, dans le conducteur écran coaxial ou dans un tube parallèle à celui-ci, au site d'opération.

13. Dispositif haute fréquence, permettant de produire un arc de plasma (5) selon l'une des revendications 6 à 12,
**caractérisé en ce que**, pour choisir différentes intensités de courant dans l'arc de plasma (5), la deuxième réactance (9b) réalisée en tant que capacité (22) est choisie de façon approprié dans un premier groupe de condensateurs (39) à l'aide d'un premier commutateur (41) et pour remplir la condition de résonance de l'inductance parallèle (19) et de la capacité efficace (18) du câble d'alimentation (10), les électrodes (4) étant raccordées à celui-ci, un condensateur approprié (28) qui est choisi à l'aide d'un deuxième commutateur (42) d'un deuxième groupe de condensateurs (40), est commuté en parallèle.

14. Dispositif haute fréquence, permettant de produire un arc de plasma (5) selon la revendication 5 ou l'une des revendications 7 à 12,
**caractérisé en ce que**, pour choisir diverses intensités de courant dans l'arc de plasma (5), la deuxième réactance réalisée en tant qu'inductance en série (21) est choisie de façon appropriée dans un groupe d'inductances (43) à l'aide d'un premier commutateur (41) et pour remplir la condition de résonance de la capacité efficace (18) du câble d'alimentation (10), les électrodes (4) étant raccordées à celui-ci, un condensateur approprié (28) qui est choisi à l'aide d'un deuxième commutateur (42) d'un groupe de condensateurs (39), est commuté en parallèle.

15. Dispositif haute fréquence, permettant de produire un arc de plasma (5) selon l'une des revendications précédentes,
**caractérisé en ce que** l'on affecte à un générateur haute fréquence (44) qui est déterminé pour d'autres applications, un transformateur (29) avec plusieurs réglages du rapport de traduction pouvant être sélectionnés, et un premier commutateur (1) étant relié à celui-ci pour sélectionner au préalable le rapport de traduction.

16. Dispositif haute fréquence, permettant de produire un arc de plasma (5) selon l'une des revendications précédentes,
**caractérisé par** une source de courant HF de haute résistance (15) et un montage de transformation en résonance (47) qui convertit la source de courant HF de haute résistance en une source de tension haute fréquence (7) avec une résistance interne de basse résistance (8).

17. Dispositif haute fréquence, permettant de produire un arc de plasma (5) selon l'une des revendications précédentes,
**caractérisé en ce que** le générateur haute fréquence (3) est formé par une source de tension haute fréquence (7) avec une forme de signal périodique, de préférence, une
forme rectangulaire, avec une résistance intérieure faible (8) qui est en particulier de l'ordre de grandeur des impédances d'ondes des câbles d'alimentation coaxiaux.

18. Dispositif haute fréquence, permettant de produire un arc de plasma (5) selon la revendication 17,
**caractérisé en ce qu'**un générateur haute fréquence (3) est formé avec une forme de signal rectangulaire par une source de tension continue (25) avec un montage complémentaire commuté en parallèle à celui-ci, de transistors de commutation (27) activés en symétrie, et ceux-ci sont commandés par un pulsateur (30) qui est synchronisé à l'aide d'un découplage de courant (26) pour mesurer le courant du circuit de résonance et la tension de départ du générateur haute fréquence ainsi formé (3) se situe au niveau de l'électrode source commune (16) des transistors de commutation (27).

19. Dispositif haute fréquence, permettant de produire un arc de plasma (5) selon l'une des revendications précédentes,
**caractérisé en ce que** le générateur haute fréquence (3) possède une fréquence propre prédéterminée et un dispositif de réglage est présent, par le biais duquel la résonance dans le montage en résonance (2) peut être régulé par réglage d'au moins l'une des deux réactances.

20. Dispositif haute fréquence, permettant de produire un arc de plasma (5) selon la revendication 19,
**caractérisé en ce que** la résonance dans le montage en résonance (2) est définie par réglage de la première réactance (9a) de telle sorte qu'un découplage de courant (26) dans le circuit électrique du montage en résonance (2) soit présent et que le courant découplé soit acheminé à un indicateur d'amplitude (32) et soit évalué dans celui-ci et un régulateur (33) destiné à régler la réactance (9a) (32) est monté en aval de l'indicateur d'amplitude, dans lequel le signal de l'indicateur d'amplitude est évalué de telle sorte que le courant de résonance et ainsi, la tension au niveau de la première réactance (9a) soient réglés à un niveau maximal.

21. Dispositif haute fréquence, permettant de produire un arc de plasma (5) selon la revendication 19 ou 20,
**caractérisé en ce que** le dispositif de réglage présente un indicateur de valeur théorique (36) pour prérégler le courant dans l'arc de plasma (5) et ainsi, le courant pour le patient.

22. Dispositif haute fréquence, permettant de produire un arc de plasma (5) selon la revendication 21 **caractérisé en ce qu'**un découplage de courant du patient (37), un indicateur de courant du patient (34) et un régulateur de courant du patient (35) sont prévus et le courant dans l'arc de plasma (5) peut être réglé par réglage de la deuxième réactance (9b) et de la source de tension haute fréquence (7) à la valeur prédéterminée par l'indicateur de valeur théorique (36).

23. Dispositif haute fréquence, permettant de produire un arc de plasma (5) selon l'une des revendications précédentes,
**caractérisé en ce qu'**au moins l'une des deux réactances (9a, 9b) est formée complètement ou partiellement par une inductance variable (19b), dont la valeur d'inductance est donnée sans inertie par prémagnétisation d'un noyau de ferrite présentant une perméabilité variable (52), la prémagnétisation étant réglée à l'aide d'un enroulement de prémagnétisation (53) parcouru par un courant de prémagnétisation (15).

24. Dispositif haute fréquence, permettant de produire un arc de plasma (5) selon la revendication 23,
**caractérisé en ce que** le courant de prémagnétisation variable (55) est réglé à l'aide d'une source de tension continue (56) variable.

25. Dispositif haute fréquence, permettant de produire un arc de plasma (5) selon l'une des revendications précédentes,
**caractérisé en ce que** la source de tension haute fréquence (7) et au moins des parties du montage en résonance (2), en particulier conjointement avec des dispositifs de régulation ou de réglage pour régler la fréquence de résonance ou régler l'intensité du courant plasmatique sont placées dans un boîtier garantissant la sécurité du patient, auquel le câble d'alimentation (10) est raccordé, l'électrode étant raccordée à celui-ci.
